# EUROPEAN PATENT APPLICATION

(11) **EP 4 509 615 A1**
(43) Date of publication of application: **19.02.2025**
(21) Application number: 23192184.2
(22) Date of filing: 18.08.2023
(51) Int. Cl.: C12Q 1/6813, C12Q 1/6818, G01N 33/532, G01N 33/533, G01N 33/542, G01N 33/58

(54) **OPTICALLY DETECTABLE LABEL AND MARKER**

(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

An optically detectable label (102, 500) is provided comprising a nucleic acid backbone (108, 504) comprising multiple attachment positions (306, 308, 506a, 506b, 506c, 506d) including at least a first attachment position and a second attachment position. The optically detectable label (102, 500) further comprises multiple labelling moieties (106a, 106b, 106c, 106d, 106e, 310, 312, 502a, 502b, 502c, 502d), including at least a first labelling moiety and a second labelling moiety, each labelling moiety (106a, 106b, 106c, 106d, 106e, 310, 312, 502a, 502b, 502c, 502d) attached to a different one of the attachment positions (306, 308, 506a, 506b, 506c, 506d) of the nucleic acid backbone (108, 504). The first labelling moiety and the second labelling moiety are configured to form an energy transfer donor and acceptor pair. Further, the nucleic acid backbone (108, 504) has at least a first spatial state (300, 500a) and a second spatial state (322, 500b), wherein in the first spatial state the attachment position to which the first labelling moiety and the attachment position to which the second labelling moiety are attached to, are in proximity in order to enable an energy transfer between the first labelling moiety and the second labelling moiety. Moreover, the nucleic acid backbone (108, 504) is in the first spatial state (300, 500a) when a first staple strand (302, 510, 512) is bound to the nucleic acid backbone (108, 504). In further aspects, a marker (100) comprising the optically detectable label (102, 500) is provided and a method for analysing a biological sample.

## Description

### Technical field

The invention relates to an optically detectable label and an optically detectable marker comprising the label and a kit comprising a plurality of optically detectable labels. In a further aspect, a method for analysing a biological sample with the marker is provided.

### Background

The ability to analyse biological samples, such as tissue sections, by proteomic, genomic and transcriptomic techniques has a rapidly progressed in recent years. The ability to provide super-resolution imaging of these samples has equally seen rapid progress.

For example, providing spatially resolved data for biological samples generally relies on marking the target proteins with optically detectable moieties. In order to detect a large number of target proteins simultaneously in a given sample, the target proteins are marked with distinguishable fluorescent markers. Further, multiplexing approaches are known in the art, which aim at increasing the number of proteins that may be distinguished by cyclically staining different sets of target proteins with the fluorescent markers.

However, the use of fluorescent markers inherently limits the number of target analytes that may be detected at any one time even when considering spectral unmixing strategies. Especially the ability to generate a large number of optically detectable labels distinguishable by their emission wavelength remains a challenge. Further, the repeated staining may degrade the sample and consequently be limiting as well. Thus, it remains an objective to provide means for optically distinguishing a large number of target analytes in biological sample at any one time.

### Summary

It is an object to provide an optically detectable label that is optically distinguishable from known fluorescent markers and a method for analysing a biological sample.

The aforementioned object is achieved by the subject-matter of the independent claims. Advantageous embodiments are defined in the dependent claims and the following description.

An optically detectable label is provided comprising a nucleic acid backbone comprising multiple attachment positions including at least a first attachment position and a second attachment position. The optically detectable label further comprises multiple labelling moieties, including at least a first labelling moiety and a second labelling moiety, each labelling moiety being attached to a different one of the attachment positions of the nucleic acid backbone. The first labelling moiety and the second labelling moiety are configured to form an energy transfer donor and acceptor pair. Further, the nucleic acid backbone has at least a first spatial state and a second spatial state, wherein in the first spatial state the attachment position to which the first labelling moiety and the attachment position to which the second labelling moiety are attached to, are in proximity in order to enable an energy transfer between the first labelling moiety and the second labelling moiety. Moreover, the nucleic acid backbone is in the first spatial state when a first staple strand is bound to the nucleic acid backbone.

By providing an optically detectable label with labelling moieties that may be brought into close proximity to form an energy transfer pair, the label may not only be distinguished by the excitation and/or emission wavelengths of its labelling moieties, but also by the ability of its labelling moieties to form the energy transfer pair.

In particular, the nucleic acid backbone may essentially consist of DNA. In particular, the first labelling moiety and the second labelling moiety are configured to form a non-radiative energy transfer donor and acceptor pair. For example, the energy transfer may be a Förster resonance energy transfer. The first staple strand may be bound to the nucleic acid backbone by hybridisation to binding sites on the nucleic acid backbone specific to the first staple strand.

In particular, in the second spatial state, when no staple strand is bound to the backbone, the attachment positions, particularly the labelling moieties, are at a distance at which the energy transfer is not possible or only possible at a reduced efficiency. Thus, the proximity of the labelling moieties in the first spatial state enables the energy transfer. In particular, the labelling moieties are at a distance in the first spatial state that enables the energy transfer with at least 0.8 efficiency.

The labelling moieties may be attached to the nucleic acid backbone covalently or by means of oligonucleotides specific to a sequence of the respective attachment position, for example.

For example, the nucleic acid backbone may be a DNA-origami comprising scaffold strands and further staple strands being configured to bind to the scaffold strands at predetermined positions to fold the scaffold strand into a predetermined shape. These DNA-origami structures may range in size from a few nanometres into the micron range. For the fabrication of such DNA origami-based structures longer DNA molecules (scaffold strands) can be folded at precisely identified positions by the further staple strands. The DNA origami may be designed to provide a self-assembly backbone of a particular predetermined shape. This enables an easy and reproducible synthesis and assembly of the backbone. The positional resolution in this case is limited by the size of a nucleotide, which is in the range of a nanometre or below. This has been exploited in the prior art to generate fluorescent standards, wherein fluorescent dyes are connected to precisely located bands on the DNA origami. These standards are known as "nanoruler" and are used for the calibration of imaging systems like confocal or super resolution microscopes (e.g. STED), for example, as disclosed by US2014/0057805 A1.

The DNA origami provides a scaffold for the labels. Preferably, the DNA origami structure comprises at least one scaffold strand and multiple further staple strands, wherein the further staple strands are complementary to at least parts of the scaffold strand and configured to bring the scaffold strand into a predetermined conformation. In particular, the strands are oligonucleotides. This enables generating backbones with predetermined two- or three-dimensional shapes that can self-assemble. Further, this enables the site-specific placement of the attachment positions on the backbone.

Alternatively, the backbone may comprise or essentially consist of DNA bricks. These DNA bricks are shorter length oligonucleotides that have overlapping hybridising stretches in order to bind to each other and assemble the backbone.

Preferably, the first attachment position and the second attachment position are at a distance on the nucleic acid backbone, particularly in any one direction along the nucleic acid backbone, at which an energy transfer efficiency of the energy transfer is 0.2 or less, preferably 0.1 or less. In particular, a distance at which the energy transfer between the first labelling moiety and the second labelling moiety does not occur. Thus, when the first staple strand is not bound, there is no energy transfer between the labelling moieties and the energy transfer is only possible when the first staple strand is bound to bring the nucleic acid backbone into the first spatial state. This ensures that the energy transfer may be readily detected only in the first spatial state of the nucleic acid backbone.

Preferably, the first labelling moiety or the second labelling moiety is a quencher moiety configured to accept the energy transfer from the other one of the first labelling moiety or the second labelling moiety. This enables efficiently determining when the labelling moieties are in close proximity. The other labelling moiety may be a fluorophore, for example. The quencher moiety may be a dark quencher, for example, resulting in a non-radiative energy transfer from an excited fluorescent dye to the quencher moiety and the quencher moiety then dissipating the energy non-radiatively, for example.

Preferably, the first labelling moiety and the second labelling moiety is a FRET pair. FRET is a Förster resonance energy transfer, or fluorescent resonance energy transfer. Thus, the first labelling moiety and the second labelling moiety form a pair of energy transfer donor and acceptor and the energy transfer is a FRET.

Preferably, the first labelling moiety and the second labelling moiety differ in at least one optical property. In particular, they differ in their emission wavelength and/or in their excitation wavelength. This enables efficiently distinguishing between the labelling moieties.

Preferably, the first staple strand comprises a cleavage site for specifically cleaving the first staple strand by means of a cleaving agent. In particular, cleaving the first staple strand enables the nucleic acid backbone to efficiently change from the first spatial sate to its second spatial state. Preferably, each staple strand has a cleavage site with a corresponding specific cleavage agent that only cleaves the cleavage site of the particular staple strand. The cleavage site may be: a phosphorothioate (PT)-modified oligonucleotide, a disulfide bond, a peptide sequence, a restriction site/restriction enzymethi, a target for CRISPR/Cas9 or a similar enzyme, a loxP site, FLP site, a UV-cleavable linker; a corresponding cleavage agent may be: myeloperoxidase (cleaves PT), iodine (cleaves PT), silver ions (cleaves PT), mercury ions (cleaves PT), a restriction enzyme, a CRISPR/Cas9 or a similar enzyme, Cre or FRT or similar recombinase, UV light.

Preferably, the label comprises at least two - preferably a plurality - of the first labelling moieties and at least two of the second labelling moieties. This enables a particularly bright label compared to an optically detectable label having only one first labelling moiety and one second labelling moiety. Particularly, each first moiety is at a first attachment position and each second moiety is at a second attachment position. The first and second attachment positions are in proximity in the first spatial state.

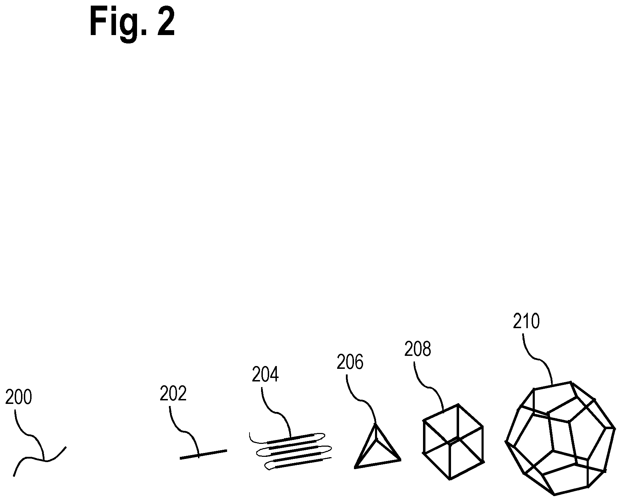

Preferably, the nucleic acid backbone comprises a first sheet-shaped structure and a second sheet-shaped structure. The first attachment position is arranged on the first sheet-shaped structure and the second attachment position is arranged on the second sheet-shaped structure. This enables a robust label. Further this enables providing a large number of first attachment positions and second attachment positions and bringing the first attachment positions and second attachment positions into proximity in the first spatial state. The sheet-shaped structures may be DNA-origami or DNA-bricks, e.g. as this is shown in Fig. 2 with the reference sign 204.

In particular, the nucleic acid backbone might comprise a first rod- or sheet-shaped structure and a second rod- or sheet-shaped structure. A plurality of first attachment positions might be provided on the first rod- or sheet-shaped structure and a plurality of second attachment positions might be provided on the second rod- or sheet-shaped structure. The first rod- or sheet-shaped structure and the second rod- or sheet-shaped structure can be brought into the first spatial state, in which the first attachment positions of the first rod- or sheet-shaped structure are in proximity with the second attachment positions of the second rod- or sheet-shaped structure, respectively, and thereby enabling energy transfers between the first labelling moieties and the second labelling moieties, in particular pairs of one of the first labelling moieties and one of the second labelling moieties. This might be accomplished for example if the first rod- or sheet-shaped structure comprises a plurality of scaffold strands, which may be located at some or on all of the loop parts of the sheet shaped structure as shown in Fig. 2 with the reference numeral 204, and the second rod- or sheet-shaped structure comprises as well a plurality of scaffold strands e.g. being located at some or on all of the loop parts of the second rod- or sheet shaped structure. Alternatively or in addition, the first and second rod- or sheet-shaped structure comprise a plurality of scaffold strands located on the face of said structures, e.g. positioned in a regular array configured to bring the first and second rod- or sheet-shaped structure into proximity upon binding of the corresponding staple strand.

If suitable staple strands are provided, which can bind one scaffold strand of the first rod- or sheep-shaped structure with the respective scaffold strand of the second rod- or sheep-shaped structure, the two rod- or sheep-shaped structures can be brought in the first state and therefore the first attachment positions of the first rod- or sheet-shaped structure can be brought in proximity with the second attachment positions of the second rod- or sheet-shaped structure.

Preferably, the number of attachment positions of the nucleic acid backbone is greater than the number of labelling moieties attached to the nucleic acid backbone. For example, the nucleic acid backbone may comprise three attachment positions and two labelling moieties. In particular, each pair of attachment positions may be brought into proximity by means of a respective staple strand. Thus, the nucleic acid backbone may have corresponding spatial states, in which the pairs of attachment positions are in proximity. This enables generating a large number of distinguishable labels.

Preferably, the label comprises a third labelling moiety, in particular, attached to one of the multiple attachment positions. The nucleic acid backbone further has at least a third spatial state, in which the third labelling is in proximity with one of the other labelling moieties. For example, the nucleic acid backbone is in the third spatial state, when a second staple strand is bound to the nucleic acid backbone.

Preferably, the third labelling moiety is configured to form an energy transfer donor and acceptor pair with at least one of the first labelling moiety and the second labelling moiety. This enables generating a large number of distinguishable labels.

In a further aspect, a kit comprising a plurality of optically detectable labels is provided. Preferably, the kit may also comprise at least the first staple strand.

In another aspect, an optically detectable marker for marking a target molecule is provided. The marker comprises the optically detectable label and an affinity reagent directly or indirectly attached to the nucleic acid backbone of the optically detectable label and configured to specifically bind to the target molecule. The optically detectable marker may further be provided as a kit with a plurality of optically detectable markers, preferably in combination with at least the first staple strand.

The marker has the same advantages as the label described above. In particular, the marker can be combined with the features of the dependent claims directed to the label.

In yet another aspect, a method for analysing a biological sample is provided. The method comprising the step of: staining the biological sample with a plurality of optically detectable markers. The marker can comprise an affinity reagent configured to specifically bind to a target molecule of the biological sample.

### Short Description of the Figures

Hereinafter, specific embodiments are described referring to the drawings, wherein:
- Figure 1: is a schematic overview of elements of an optically detectable marker comprising an optically detectable label,
- Figure 2: schematically shows various nucleic acid backbones of the optically detectable label of the marker according to Fig. 1,
- Figure 3: schematically shows the optically detectable label in various spatial states,
- Figure 4: schematically shows the optically detectable label comprising a quencher moiety in various spatial states,
- Figure 5: schematically shows the optically detectable label comprising four labelling moieties in various spatial states, and
- Figure 6: schematically shows a plurality of optically detectable labels.

### Detailed Description

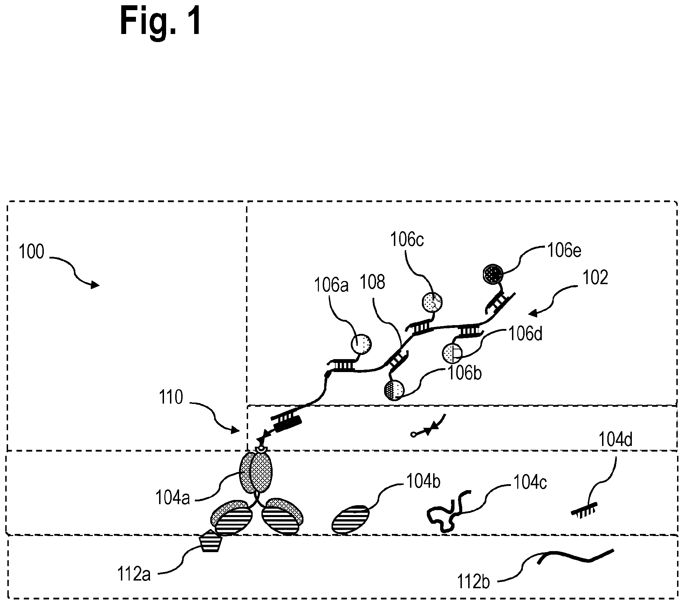

Figure 1 is a schematic overview of elements of an optically detectable marker 100 comprising an optically detectable label 102 and an affinity reagent 104a.

The label 102 may comprise several labelling moieties 106a, 106b, 106c, 106d, 106e. The labelling moieties 106a to 106e may be fluorophores, for example. At least two of the labelling moieties 106a to 106e are configured to form an energy transfer donor and acceptor pair. In particular, the energy transfer may be a non-radiative energy-transfer, such as fluorescence resonance energy transfer or Förster resonance energy transfer (FRET).

The labelling moieties 106a to 106e are attached to a nucleic acid backbone 108. In particular, the nucleic acid backbone 108 may essentially consist of at least one nucleic acid strand, such as DNA. Alternatively, the nucleic acid backbone 108 may be DNA-nanotechnology based, such as a DNA Origami.

The labelling moieties 106a to 106e are attached to the backbone 108 at attachment positions via short oligonucleotides that have a sequence that is at least partially complementary to a part of the nucleic acid backbone 108. Alternatively, the labelling moieties 106a to 106e may be attached to the nucleic acid backbone 108 covalently, in particular, to a sugar-phosphate chain of the nucleic acid backbone 108.

The attachment positions, that the labelling moieties 106a to 106e are attached to, are at a distance of each other in any one direction along the backbone 108 at which an energy transfer between adjacent labelling moieties 106a to 106e has an efficiency of 0.2 or less, preferably 0.1 or less, or more preferably at which no energy transfer between adjacent labelling moieties 106a to 106e may take place. Thus, the labelling moieties 106a to 106e are at a corresponding distance, in the state of the backbone 108 as shown in Figure 1.

The label 102 may be attached to the affinity reagent 104a via a linker 110. The linker may comprise an affinity interactor pair, such as biotin and streptavidin, for attaching the label 102 to the affinity reagent 104a. Alternatively or in addition, the linker 110 may comprise an oligonucleotide that is complementary to a part of the backbone 108 of the label 108. Further, the linker 110 may additionally comprise a cleavage site (depicted by the two black triangles of the linker 110 shown on the right-hand side) for removing the label 102 from the affinity reagent 104a.

The affinity reagent 104a may alternatively be an antibody fragment 104b, an aptamer 104c, or a linear oligonucleotide 104d. The affinity reagents 104a to 104d are configured to specifically bind to a target analyte 112a in a biological sample, for example. The target analyte 112a may be a protein for example, to which at least the affinity reagents 104a to 104c may bind due to their complex secondary, tertiary, and/or quaternary structure. Alternatively, the target analyte may be a mRNA 112b, for example, to which the affinity reagent 104d may bind due to its nucleotide sequence, or primary structure, via hybridisation.

Thus, the affinity reagent 104a to 104d of the marker 100 may bind to the target analyte 112a, 112b of the biological sample. The label of the marker 100 may then be optically detected, for example, by means of a (fluorescence) microscope in order to identify and/or locate the target analyte 112a, 112b within the biological sample.

Figure 2 schematically shows various alternatives of the nucleic acid backbone 108 of the optically detectable label 102 of the marker 100. For example, the backbone 108 may be a linear nucleic acid strand 200, in particular comprising between 100 and 300 nucleotides. Alternatively, the backbone may be a DNA-Origami 202, 204, 206, 208, 210 with different geometries. In particular, the DNA-origami 202 is linear or rod-like. The DNA-origami 204 is sheet-like, which may be a large linear DNA molecule or an assembly of multiple DNA molecules. Sheet-like geometries may increase the number of available attachment positions substantially. Further geometries are possible, for example, a tetrahedral DNA-origami 206, cubic DNA-origami 208, or a polyhedral DNA-origami 210.

In particular, the backbone 108 being DNA-origami based, allows generating predetermined, stable two- and three-dimensional shapes. Further, this allows generating a plurality of attachment positions at predetermined positions along the backbone 108. The attachment positions are nucleotide sequences stretches, that are unique and allow the hybridisation of complementary oligonucleotides, for example, to attach the labelling moieties 106a to 106e to the backbone 108 at these predetermined positions. Alternatively, the attachment positions may be functionalised nucleotides that are incorporated into the backbone 108 and to which the labelling moieties 106a to 106e are attached covalently.

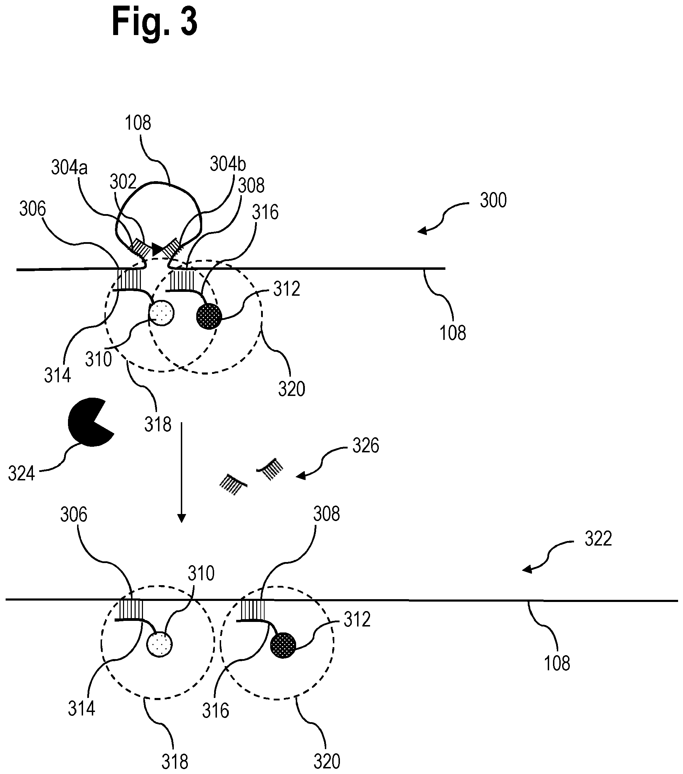

Figure 3 schematically shows an optically detectable label in various spatial states. In a first spatial state 300 of the optically detectable label, in particular of the backbone 108, a staple strand 302 is bound to binding sites 304a, 304b of the nucleic acid backbone 108 of the label. Thus, due to the binding of the staple strand 302 to the backbone 108, the backbone 108 is brought into the first spatial state 300. In particular, the backbone 108 may be folded when the staple strand 302 is attached. In order for the staple strand 302 to bind to the binding sites 304a, 304b, the staple strand comprises sequence stretches complementary to the binding sites 304a, 304b. Preferably, the binding sites 304a, 304b are unique such that only the staple strand 302 may bind to the binding sites 304a, 304b.

The nucleic acid backbone 108 comprises attachment positions 306, 308 at which labelling moieties 310, 312, such as fluorophores, are attached via complementary oligonucleotides 314, 316 to the backbone 108. The respective labelling moieties 310, 312 may form an energy transfer donor and acceptor pair. In the first spatial state 300 the attachment positions 306, 308 are in proximity that enables an energy transfer between the labelling moieties 310, 312. Thus, in the first spatial state 300 also the labelling moieties 310, 312 are at a corresponding proximity that allows an energy transfer between the labelling moieties 310, 312. In particular, in the first spatial state 300, the labelling moieties 310, 312 are within each other's interaction radii 318, 320. Preferably, when the labelling moieties 310, 312 are within each other's interaction radii 318, 320 the energy transfer efficiency between the energy transfer donor and acceptor pair is at least 0.8.

For example, the labelling moiety 310 is the energy transfer donor and the labelling moiety 312 is the energy transfer acceptor. When the labelling moiety 310 is excited by excitation light of an excitation wavelength, instead of emitting fluorescent emission light of an excitation wavelength, the energy transfer, in particular non-radiative energy transfer, from the donor labelling moiety 310 to the acceptor labelling moiety 312 causes the acceptor labelling moiety 312 to accept the energy and thereby excite the acceptor labelling moiety 312. Thus, the acceptor labelling moiety 312 emits fluorescent emission light of an excitation wavelength. Preferably, the optical properties of the labelling moieties 310, 312 are different. Specifically, the labelling moieties 310, 312 may differ in at least one of their excitation wavelength, or their emission wavelength. This ensures that the labelling moieties 310, 312 can be distinguished in an optical readout.

The label further has a second spatial state 322, in which the staple strand 302 is not bound to the backbone 108. In the second spatial state 322, the attachment positions 306, 308 are preferably at a distance that does not allow an energy transfer between the labelling moieties 310, 312, or only with an efficiency of 0.2 or less. Thus, in the second spatial state 322 also the labelling moieties 310, 312 are at a corresponding proximity that does not allow the labelling moieties 310, 312 to form an energy transfer donor and acceptor pair. In particular, in the second spatial state 322, the labelling moieties 310, 312 are not within each other's interaction radii 318, 320.

In the second spatial state 322 when the labelling moiety 310 is excited by excitation light of the excitation wavelength, the labelling moiety 310 emits fluorescent emission light of the excitation wavelength. No energy is transferred from the labelling moiety 310 to the labelling moiety 312. Instead, the labelling moiety 312 may be separately excited by excitation light and upon excitation emit fluorescent emission light. Preferably, the optical properties of the labelling moieties 310, 312 are different, as detailed above. Thus, by providing the labelling moiety 312 at the attachment position 308, the apparent fluorescence emission properties from the label according to Figure 3 may be changed depending on the spatial state 300, 322 of the backbone 108. Thus, when the staple strand 302 is not bound to the backbone 108 or when the staple strand 302 is removed from the backbone 108, the label is in its second spatial state 322. In order to irreversibly remove the staple strand 302, the staple strand may comprise a cleavage site specifically cleavable by a cleavage agent. The cleavage site may be a restriction site, for example, cleavable by a particular restriction enzyme 324 that preferably does not cleave the backbone 108. The staple strand 302 may consequently be removed from the backbone 108 by addition of the restriction enzyme resulting in staple strand fragments 326.

Alternatively, the staple strand 302 may be reversibly removed from the backbone 108 by providing environmental conditions in which the staple strand 302 only transiently binds to the binding sites 304a, 304b of the backbone 108. In this case, the label is continuously switching between the first spatial state 300 and the second spatial state 322, depending on whether the staple strand 302 is bound to the backbone 108 or not. This may cause blinking of the labelling moiety 312 when the labelling moiety 310 is excited with excitation light, when the backbone 108 switches between the first spatial state 300 and the second spatial state 322. The blinking may be used to perform super resolution imaging by single molecule localization microscopy. The environmental conditions may be a particular temperature or a temperature profile the label is at. Alternatively or additionally, the environmental conditions may be a buffer concentration or type.

Generally, removal of the staple strand 302 may be performed by a variety of enzymatic, chemical or physical methods. Alternatively, removal may be based on strand displacement and brought about by the addition of suitable capture strands that are complementary to the staple strand 302. Cleavage sites may in particular be phosphorothioate (PT) modified oligonucleotides, disulfide bridge modification, peptide modification, other organic linkers that can be incorporated into modified oligonucleotides. Likewise, cleaving agents such iodine, silver nitrate, mercury salts, TCEP, hydrogen peroxide are available that work with corresponding cleavage sites, e.g. iodine, silver ions, mercury ions may be used cleave PT, TCEP may be used to cleave disulfide brigdes. Further UV light and UV cleavable linkers can be used. Further, the staple strand 302 may comprise a peptide as a cleavage site and said peptide may be sensitive to an enzyme like Caspase or a protease.

Alternatively or in addition, the staple strand 302 and the nucleic acid backbone 108 may have differential sensitivity to a nuclease like DNase I. This may be achieved by using a nucleic acid backbone 108 comprising an artificial nucleic acid (XNA) that is nuclease-resistant. Like for example, the nucleic acid backbone 108 may comprise a threose nucleic acid (TNA) and the staple strand 302 may comprise DNA. TNA was found to exhibit high resistance to DNasel. DNasel has been successfully used in the prior art to perform cyclic staining using both antibody- DNA oligo-based label conjugates as well as smFISH-DNA oligo-based label conjugates probes. In this case, bound staple strands can be easily removed by digesting. Alternatively or in addition, a staple strand 108 may comprise at least one restriction site. This allows enzymatic removal by using DNasel or by using a restriction enzyme.

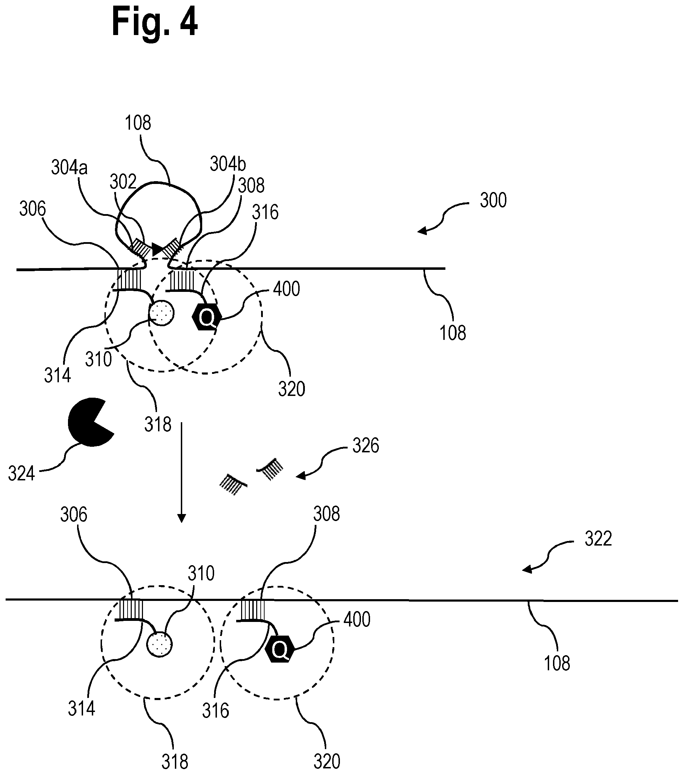

Figure 4 schematically shows an optically detectable label comprising a quencher moiety 400 as a labelling moiety and comprising the nucleic acid backbone 108 in the first spatial state 300. As detailed above for Figure 3, in the first spatial state 300 the staple strand 302 is bound to the binding sites 304a, 304b of the nucleic acid backbone 108. Thus, due to the binding of the staple strand 302 to the backbone 108, the backbone 108 is brought into the first spatial state 300. The label shown in Figure 4 comprises the labelling moiety 310 at the attachment position 306. However, at the attachment position 308 the quencher moiety 400 is attached to the backbone 108 via the complementary oligonucleotide 316. The quencher moiety 400 may be a fluorescent quencher such as TAMRA or a non-fluorescent quencher or dark quencher such as Dabcyl.

Thus, in case the quencher moiety 400 is a dark quencher, as detailed for Figure 3 and the labelling moieties 310, 312, the labelling moiety 310 and the quencher moiety 400 may similarly form an energy transfer donor and acceptor pair. For example, when the labelling moiety 310 and the quencher moiety 400 are within each other's interaction radii 318, 320 when the backbone 108 is in its first spatial state 300, the excitation of the labelling moiety 310 may cause an energy transfer to the quencher moiety 400 instead of an emission by the labelling moiety 310. In contrast to the labelling moiety 312, the quencher moiety 400 may dissipate the transferred energy as heat instead of fluorescent emission. Thus, in the first spatial state 300 of the backbone 108 no fluorescent emission can be detected since the excitation of the labelling moiety 310 results in an energy transfer to the quencher 400, which does not emit fluorescent light upon the energy transfer. However, in case the backbone 108 is in the second spatial state 322 and the labelling moiety 310 and the quencher moiety 400 are not within each other's interaction radii 318, 320, the labelling moiety 310 emits fluorescence light upon excitation. Thus, by providing the quencher moiety 400 at the attachment position 308, the fluorescence emission of the labelling moiety 310 at the attachment position 306 can be switched on and off depending on the spatial state 300, 322 of the backbone 108.

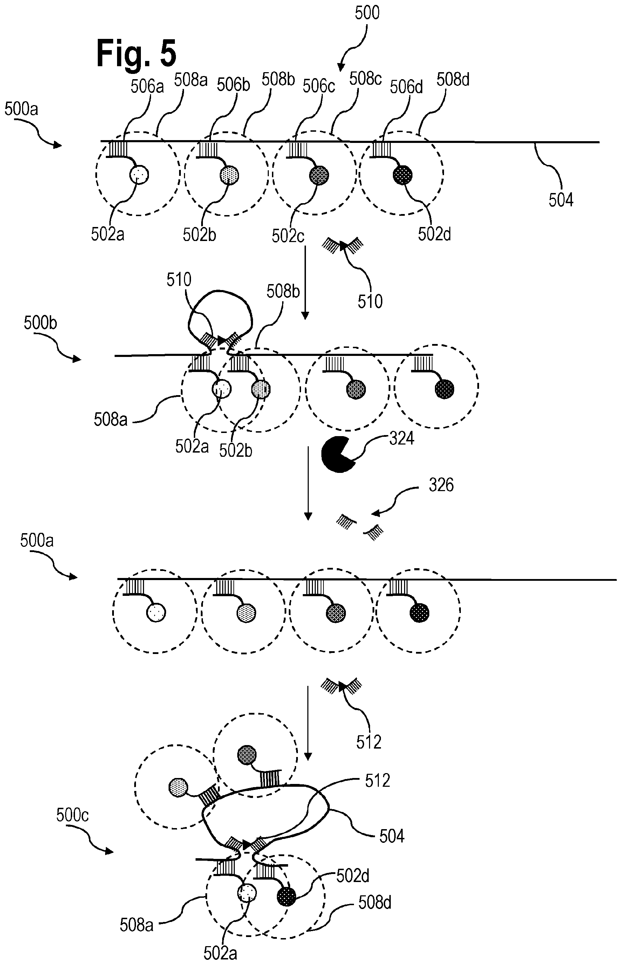

Figure 5 schematically shows an optically detectable label 500 comprising four labelling moieties 502a, 502b, 502c, 502d in various spatial states attached to a nucleic acid backbone 504 at attachment positions 506a, 506b, 506c, 506d. At least the labelling moieties 502a and 502b as well as 502a and 502d are configured to form energy transfer donor and acceptor pairs.

The nucleic acid backbone 504 has at least three spatial states. In a first spatial state, indicated by the reference sign 500a, none of the labelling moieties 502a to 502d are within each other's interaction radii 508a, 508b, 508c, 508d. In this case, time sequentially or simultaneously excitation of each of the labelling moieties 502a to 502d results in their individual emission.

In a second spatial state of the nucleic acid backbone 504, indicated by reference sign 500b, a first staple strand 510 is bound to binding sites of the backbone 504. This causes the attachment positions 506a, 506b to be in proximity such that the labelling moieties 502a, 502b are in corresponding proximity and within each other's interaction radii 508a, 508b. Thus, in case the labelling moiety 502a is excited by excitation light an energy transfer between the labelling moieties 502a, 502b causes the labelling moiety 502b to emit emission light.

In a third spatial state of the nucleic acid backbone 504, indicated by reference sign 500c, a second staple strand 512 is bound to binding sites of the backbone 504. This causes the attachment positions 506a, 506d to be in proximity such that the labelling moieties 502a, 502d are in corresponding proximity and within each other's interaction radii 508a, 508d. Thus, in case the labelling moiety 502a is excited by excitation light an energy transfer between the labelling moieties 502a, 502d causes the labelling moiety 502d to emit emission light.

In both, the second and third spatial state 500b, 500c, the respective labelling moieties 502c, 502d or 502b, 502c are not in proximity to an energy transfer partner and may therefore be excited to emit light at their native wavelengths.

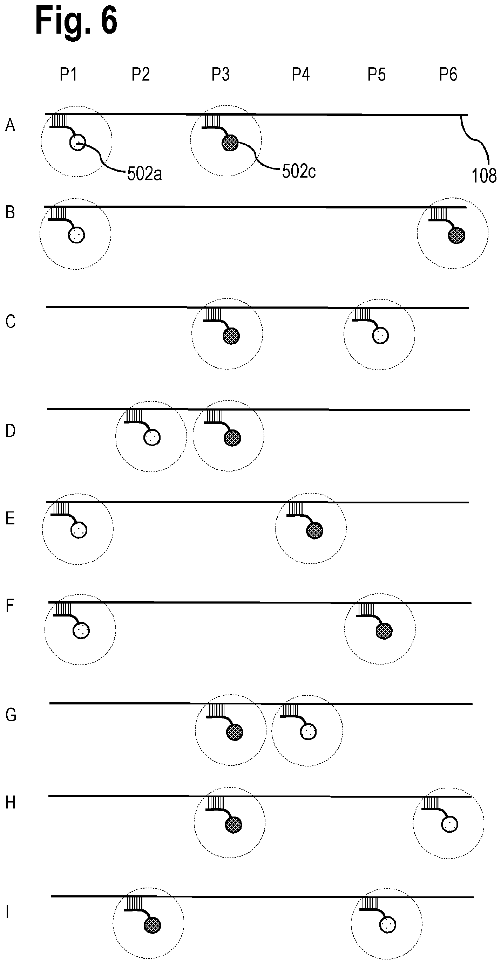

Figure 6 shows schematically a plurality of optically detectable labels comprising 9 labels referred to as A, B, C, D, E, F, G, H, I, with each of the labels comprising the nucleic acid backbone 108 with 6 attachment positions P1, P2, P3, P4, P5, P6. Each label comprises one of each of the labelling moieties 502a, 502c attached to a different one of the attachment positions. The labelling moieties 502a, 502c are configured to form an energy transfer donor and acceptor pair. Further, the labels differ from each other by the specific attachment positions the labelling moieties 502a, 502c are attached to.

In the spatial state the labels are shown in Figure 6, the labelling moieties 502a, 502c are not in proximity for an energy transfer between them to occur. Thus, the labelling moieties 502a, 502c may not be distinguished from each other based on their excitation or emission wavelength. However, each pair of the attachment positions P1, P2, P3, P4, P5, P6 may be brought into a spatial state in which the labelling moieties 502a, 502c are in proximity to form an energy transfer donor and acceptor pair by adding suitable staple strands to the labels A, B, C, D, E, F, G, H, I. By iteratively adding and removing these staple strands, for example by cleaving, each pair of attachment positions may be optically readout. The pair of positions that have the labelling moieties 502a, 502c may be identified by a change in the optical properties such as emission wavelengths due to the energy transfer between the labelling moieties 502a, 502c. Thus, the attachment positions to which the labelling moieties 502a, 502c are attached to may be determined and based on that the labels may be distinguished from each other. The above similarly applies in case one of the labelling moieties 502a, 502c is a quencher moiety.

In a method for analysing a biological sample a plurality of optically detectable labels disclosed herein may be combined with markers to form a plurality of optically detectable markers. The optically detectable markers may be used for staining a biological sample. This means introducing the optically detectable markers into the biological sample and allowing them to attach to their respective target analytes by means of their respective affinity reagents. The markers, in particular the labels, may be optically readout, for example by means of a microscope, and the target analytes within the sample identified and/or localised. As explained above, staple strands may be added in order to bring into proximity the attachment positions and identify the attachment positions that carry labelling moieties. Similarly, this may be carried out in an iterative process of adding and removing staple strands, as well as generating an optical readout.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100: Optically detectable marker
- 102, 500: Optically detectable label
- 104a, 104b, 104c, 104d: Affinity Reagent
- 106a,106b,106c,106d,106e, 310,312,502a,502b,502c, 502d: Labelling moiety
- 108, 504: Nucleic acid backbone
- 110: Linker
- 112a, 112b: Target analyte
- 200: Linear nucleic acid backbone
- 202, 204, 206, 208, 210: DNA-Origami backbone
- 300, 500a: First spatial state
- 302, 510, 512: Staple strand
- 304a, 304b: Staple strand binding site
- 306, 308, 506a, 506b, 506c, 506d: Attachment position
- 314, 316: Complementary oligonucleotide
- 318, 320, 508a, 508b, 508c, 508d: Labelling moiety interaction radius
- 322, 500b: Second spatial state
- 324: Restriction enzyme
- 326: Staple strand fragments
- 400: Quencher labelling moiety
- 500c: Third spatial state

## Claims

1. An optically detectable label (102, 500) comprising:
a nucleic acid backbone (108, 504) comprising multiple attachment positions (306, 308, 506a, 506b, 506c, 506d) including at least a first attachment position and a second attachment position,
multiple labelling moieties (106a, 106b, 106c, 106d, 106e, 310, 312, 502a, 502b, 502c, 502d), including at least a first labelling moiety and a second labelling moiety, each labelling moiety (106a, 106b, 106c, 106d, 106e, 310, 312, 502a, 502b, 502c, 502d) attached to a different one of the attachment positions (306, 308, 506a, 506b, 506c, 506d) of the nucleic acid backbone (108, 504), wherein the first labelling moiety and the second labelling moiety are configured to form an energy transfer donor and acceptor pair,
wherein the nucleic acid backbone (108, 504) has at least a first spatial state (300, 500a) and a second spatial state (322, 500b), wherein in the first spatial state (300, 500a) the attachment position to which the first labelling moiety and the attachment position to which the second labelling moiety are attached to are in proximity in order to enable an energy transfer between the first labelling moiety and the second labelling moiety, and wherein the nucleic acid backbone (108, 504) is in the first spatial state (300, 500a) when a first staple strand (302, 510, 512) is bound to the nucleic acid backbone (108, 504).

2. The optically detectable label according to claim 1, wherein the first attachment position and the second attachment position are at a distance on the nucleic acid backbone (108, 504) at which an energy transfer efficiency is 0.2 or less.

3. The optically detectable label according to one of the preceding claims, wherein the first labelling moiety or the second labelling moiety is a quencher moiety (400) configured to accept the energy transfer from the other one of the first labelling moiety or the second labelling moiety.

4. The optically detectable label according to one of the preceding claims, wherein the first labelling moiety and the second labelling moiety is a FRET pair.

5. The optically detectable label according to one of the preceding claims, wherein the first labelling moiety and the second labelling moiety differ in at least one optical property.

6. The optically detectable label according to one of the preceding claims, wherein the first staple strand (302, 510, 512) comprises a cleavage site for specifically cleaving the first staple strand (302, 510, 512) by means of a cleaving agent (324).

7. The optically detectable label according to one of the preceding claims, comprising at least two of the first labelling moieties and at least two of the second labelling moieties.

8. The optically detectable label according to one of the preceding claims, wherein the nucleic acid backbone (108, 504) comprises a first sheet-shaped structure and a second sheet-shaped structure, wherein the first attachment position is arranged on the first sheet-shaped structure and the second attachment position is arranged on the second sheet-shaped structure.

9. The optically detectable label according to one of the preceding claims, wherein the number of attachment positions (306, 308, 506a, 506b, 506c, 506d) of the nucleic acid backbone (108, 504) is greater than the number of labelling moieties (106a, 106b, 106c, 106d, 106e, 310, 312, 502a, 502b, 502c, 502d) attached to the nucleic acid backbone (108, 504).

10. The optically detectable label according to one of the preceding claims, comprising a third labelling moiety.

11. The optically detectable label according to claim 10, wherein the third labelling moiety is configured to form an energy transfer donor and acceptor pair with at least one of the first labelling moiety and the second labelling moiety.

12. A kit comprising a plurality of optically detectable labels (102, 500) according to one of the preceding claims.

13. An optically detectable marker (100) for marking a target molecule comprising:
the optically detectable label (102, 500) according to one of the preceding claims 1 to 12, and
an affinity reagent (104a, 104b, 104c, 104d) directly or indirectly attached to the nucleic acid backbone (108, 504) of the optically detectable label (102, 500) and configured to specifically bind to the target molecule.

14. A method for analysing a biological sample, comprising the step of:
staining the biological sample with a plurality of optically detectable markers (100) according to claim 13.

15. The method according to claim 14, further comprising the step of generating a first optical readout of the stained biological sample.

16. The method according to claim 15, further comprising the step of adding or removing at least the first staple strand (302, 510, 512) after the generating the first optical readout and subsequently generating a second optical readout.

17. The method according to claim 16, wherein removing the first staple strand includes either adding to the biological sample a capture strand complementary to the first staple strand (302, 510, 512) or adding a cleavage agent (324) configured to specifically cleave the cleavage site of the first staple strand (302, 510, 512).
